(19) **Europäisches Patentamt**
**European Patent Office**
**Office européen des brevets**

(11) **EP 1 223 980 B1**

(12) **EUROPÄISCHE PATENTSCHRIFT**

(45) Veröffentlichungstag und Bekanntmachung des Hinweises auf die Patenterteilung:
**21.05.2003 Patentblatt 2003/21**

(21) Anmeldenummer: **00972422.0**

(22) Anmeldetag: **25.10.2000**

(51) Int Cl.⁷: **A61K 39/395**, A61P 35/00, A61K 48/00

(86) Internationale Anmeldenummer:
**PCT/AT00/00281**

(87) Internationale Veröffentlichungsnummer:
**WO 01/030381 (03.05.2001 Gazette 2001/18)**

(54) **VERWENDUNG VON CSF-1-INHIBITOREN**

USE OF CSF-1 INHIBITORS

UTILISATION D'INHIBITEURS CSF-1

(84) Benannte Vertragsstaaten:
**AT BE CH CY DE DK ES FI FR GB GR IE IT LI LU MC NL PT SE**

(30) Priorität: **28.10.1999 AT 181399**

(43) Veröffentlichungstag der Anmeldung:
**24.07.2002 Patentblatt 2002/30**

(73) Patentinhaber:
• **Hofbauer, Reinhold**
**1210 Wien (AT)**
• **Aharinejad, Seyedhossein**
**1130 Wien (AT)**

(72) Erfinder: **AHARINEJAD, Seyedhossein**
**A-1130 Wien (AT)**

(74) Vertreter: **Alge, Daniel, Mag. Dr. rer.nat. et al**
**Sonn & Partner Patentanwälte**
**Riemergasse 14**
**1010 Wien (AT)**

(56) Entgegenhaltungen:
• **HARAN-GHERA NECHAMA ET AL: "Increased circulating colony-stimulating factor-1 (CSF-1) in SJL/J mice with radiation-induced acute myeloid leukemia (AML) is associated with autocrine regulation of AML cells by CSF-1." BLOOD, Bd. 89, Nr. 7, 1997, Seiten 2537-2545, XP002165343 ISSN: 0006-4971**
• **KESHAVA N ET AL: "Blocking of colony stimulating factor expression with antisense RNA in breast and ovarian cancer epithelial cells leads to programmed cell death." PROCEEDINGS OF THE AMERICAN ASSOCIATION FOR CANCER RESEARCH ANNUAL, Bd. 38, 1997, Seite 500 XP002165344 Eighty-eighth Annual Meeting of the American Association for Cancer Research;San Diego, California, USA; April 12-16, 1997, 1997 ISSN: 0197-016X**
• **MENETRIER-CAUX C G MONTMAIN ET AL: "Inhibition of the differentiation of dendritic cells from cD34+ progenitors by tumor cells: Role of interleukin-6 and macrophage colony-stimulating factor." BLOOD, Bd. 92, Nr. 12, 15. Dezember 1998 (1998-12-15), Seiten 4778-4791, XP002165345 ISSN: 0006-4971**

**Beschreibung**

Die Erfindung betrifft die Verwendung von Inhibitoren der CSF-1-Aktivität.

Der "Koloniestimulierungsfaktor 1" ("Colony Stimulating Factor 1"; "CSF-1") ist ein Zytokin, welches in der Lage ist, vor allem Makrophagen-Kolonien zu bilden. Nativer CSF-1 ist ein glykosyliertes Dimer, wobei im Menschen verschiedene Formen dieses Moleküls mit verschiedener Länge und verschiedenem Molekulargewicht vorliegen. Beispielsweise ist bekannt, dass die zwei Hauptformen von CSF-1 mit 224 bzw. 522 Aminosäuren durch alternatives Splicing gebildet werden. Es ist weiters bekannt, dass die Minimallänge dieses Faktors etwa 150 Aminosäuren beträgt. Weiters kann auch CSF-1 in verschiedenen Glykosylierungsmustern auftreten, die je nach physiologischem Zustand spezifisch oder gewebespezifisch sind.

CSF-1 wurde zur Überwindung von Immunsuppression bei Patienten eingesetzt, die beispielsweise durch Chemotherapie bedingt war. Weitere Anwendungen betrafen die Behandlung oder Verhinderung von bakteriellen, viralen oder Pilzinfektionen, die Stimulierung von weißen Blutzellen und die Unterstützung der Wundheilung.

Weiters wurde CSF-1 auch zur Behandlung von Tumorerkrankungen verwendet (US 5 725 850) und zwar nicht nur als Unterstützung in immunsupprimierten Tumorpatienten, sondern zur direkten Abtötung von Tumorzellen. Hierbei wurde festgestellt, dass vor allem Sarkoma-Tumorzellen durch Gabe von CSF-1 abgetötet werden können (US 5 104 650).

Die Antitumorwirkung von CSF-1 ist jedoch im Stand der Technik nicht unumstritten, so berichten Anderson et al. (Gynecol. Oncol. 74(2) (1999), 202-207), dass weder CSF-1 noch dessen Rezeptor eine Rolle in der Pathogenese von uterinären Sarkomas haben. Andererseits ist bekannt, dass sowohl CSF-1 als auch dessen Rezeptor für endometrische Adenokarzinomas mit der Turmorprogression korrelieren. Schließlich konnte in CSF-1-defizienten und Makrophagen-defizienten Mäusen ein vermindertes Tumorwachstum bei einem speziellen Tumor (Lewis Lung-Karzinoma) festgestellt werden, jedoch starben die CSF-1-defizienten Mäuse trotz des verminderten Tumorwachstums schneller als die Tumor-tragenden Kontrollmäuse (Nowicki et al., Int. J. Cancer 65 (1996), 112-119). Es wurde vermutet, dass diese verminderte Lebenserwartung auch auf die massive Nekrosebildung in den CSF-1-defizienten Mäusen zurückzuführen war.

In Haran-Ghera et al. (Blood 89(7) (1997), S.2537-2545) wird die Verwendung von anti-CSF-1-Antikörpern und anti-CSF-1-Rezeptor-Antikörpern zur Hemmung der Koloniebildung von Tumorzellen in vitro offenbar. Da CSF-1 und CSF-1-Rezeptor in fast allen Leukämien induziert werden, wird postuliert, dass CSF-1 und CSF-1-Rezeptor Mittel sind, die sich wie Markermoleküle für Leukämietumorzellen verhalten.

In Keshava et al. (Proc.Am.Assoc.Canc.Res.Annual 38 (1997), S.500) wurde die Rolle von CSF-1 und dessen Rezeptor in Brust-, Eierstock- und anderen Krebsarten untersucht. Es wird beschrieben, dass der Makrophagen-CSF als autokriner Wachstumsfaktor in Brust- und Eierstockkrebs wirkt.

Demgemäß blieb zwar die Rolle von CSF-1 als Antitumormittel durchaus umstritten, eine negative Wirkung von CSF-1 auf die Behandlung von Tumoren wurde bislang im Stand der Technik aber nicht diskutiert oder für möglich gehalten.

Aufgabe der vorliegenden Erfindung ist die Bereitstellung eines Mittel zur Behandlung von Tumorpatienten, insbesondere unter Einbeziehung der Rolle von CSF-1 bei Tumoren.

Diese Aufgabe wird erfindungsgemäß gelöst durch die Verwendung von Verbindungen, die CSF-1-Aktivität inhibieren, zur Herstellung eines Mittels zur Behandlung von Tumorerkrankungen. Es hat sich im Zuge der vorliegenden Erfindung überraschenderweise herausgestellt, dass CSF-1 - entgegen den bislang im Stand der Technik vorgeschlagenen Wirkungen - selbst keine Antitumorwirkung aufweist, sondern dass das Tumorwachstum durch Gabe von Verbindungen, die CSF-1 oder dessen Rezeptor inhibieren, verzögert oder unterbunden werden kann und dies zu einer erhöhten Überlebensrate führt. Es war zwar im Stand der Technik durchaus bekannt, dass CSF-1 in einigen Tumoren mit dem Fortschritt des Tumorwachstums korreliert, bislang nahm man jedoch an, dass dieser Gehalt an CSF-1 und CSF-1-Rezeptor keinen Einfluss auf das Tumorwachstum haben würde, im Gegenteil, man ging im Stand der Technik davon aus, dass erhöhte CSF-1-Produktion zu einem Rückgang von Tumoren führte. So wird im US-Patent 5 725 850 zwar offenbart, dass erhöhte CSF-1-Konzentrationen zu einer Stimulierung von Makrophagen, die Maus-Sarkoma TU5-Zellen abtöteten, herangezogen werden kann, es wird aber auch erwähnt, dass diese Aktivität eigentlich nur dann tatsächlich effektiv ist, wenn CSF-1 in Kombination mit Interleukin-2, IFN-$\alpha$, IFN-$\beta$ oder IFN-$\gamma$ angewendet wird. Möglicherweise könnte daher dieser Sarkoma-Abtötungseffekt der im Stand der Technik berichtet wird, auf die zusätzlichen Lymphokine, die mit CSF-1 verabreicht worden sind, zurückzuführen gewesen sein.

Im Gegensatz dazu wurde im Rahmen der vorliegenden Erfindung erkannt, dass die Gabe von CSF-1 oder CSF-1-Rezeptor-inhibierenden Substanzen tatsächlich einen Antitumoreffekt aufweisen. Dies steht im Gegensatz zur bisher im Stand der Technik verbreiteten Lehre.

Der einzige Effekt, der bislang - nach Kenntnis der vorliegenden Erfindung - in Richtung eines negativen Effektes von CSF-1 in Zusammenhang mit Tumorerkrankungen hindeutet, war bislang ein behindertes Tumorwachstum in CSF-1-defizienten Makrophagen-defizienten Mäusen. Hierbei wurde auf die Rolle von CSF-1-abhängigen Makrophagen bei der Bildung von Tumorstroma hingewiesen (siehe Nowicki et al.), indem geschlossen wird, dass das LLC-Tumorwachstum in CSF-1-defizienten Mäusen nicht durch die Abwesenheit von CSF-1-abhängigen Makrophagen erleichtert wird (wie eigentlich auf Grund der bislang im Stand der Technik beschriebenen Antitumorwirkung von CSF-1 selbst erwartet hätte werden können). Hier wurde weiters auch auf die signifikanten Antitumoreffekte, die bei der in vivo-Behandlung von Mäusen mit CSF-1 gezeigt werden konnten, hingewiesen. Es zeigte sich zwar bei CSF-1-defizienten Mäusen, denen ein LLC-Tumor eingepflanzt wurde, dass das Tumorwachstum nicht gegenüber normalen Mäusen erhöht war, sondern tatsächlich die defizienten Mäuse wenig Stromagewebe hatten. Die LLC-Tumoren in diesen Tieren waren wesentlich nekrotischer; darauf wurde auch das reduzierte Wachstum zurückgeführt. Jedenfalls starben die CSF-1-defizienten Mäuse früher als die Tumor-erkrankten Kontrollmäuse. Nowicki et al. hielten zunächst einmal fest, dass der LLC-Tumor kein repräsentativer Tumor ist, um die Rolle von CSF-1 in der natürlichen Antitumor-Immunität zu belegen. Dieser Tumor wurde im Nowicki et al.-Modell lediglich deshalb verwendet, weil er sowohl in Kontrollmäusen als auch in CSF-1-Mäusen reproduzierbar gewachsen ist.

Ebenfalls wird von Nowicki et al. festgehalten, dass die mit CSF-1 defizienten Mäusen erhaltenen Daten nicht gegen die Hypothese sprechen, dass CSF-1-abhängige Makrophagen eine wichtige Rolle in der induzierten Antitumorantwort spielen, besonders wenn ein Stimulus mit exogenem CSF-1 erfolgt, wie dies im Stand der Technik berichtet worden ist.

Tatsächlich stellt sich aber im Rahmen der vorliegenden Erfindung heraus, dass nicht die Gabe von CSF-1 selbst eine Antitumorantwort auslöst bzw. zur Behandlung von Tumorerkrankungen eingesetzt werden kann, sondern eine effiziente Tumorbehandlung durch Inhibierung von CSF-1-Aktivität erfolgen kann.

Demgemäß betrifft die vorliegende Erfindung die Verwendung von Inhibitoren von CSF-1-Aktivität zur Herstellung eines Arzneimittels zur Behandlung von Tumorerkrankungen. Das erfindungsgemäße Mittel zur Behandlung von Tumorerkrankungen, enthaltend Inhibitoren von CSF-1-Aktivität steht somit im Gegensatz zur vorherrschenden Lehre, in welcher CSF-1 selbst eher ein Antitumor-effekt zugestanden wurde, oder aber zumindest eine neutrale Rolle von CSF-1 bei den meisten Tumorerkrankungen vermutet wurde.

Bei der vorliegenden Erfindung wird in einem Verfahren zur Behandlung von Tumorerkrankungen eine effiziente Dosis von Inhibitoren von CSF-1-Aktivität an Tumorpatienten verabreicht.

Die Art und Weise, in welcher die CSF-1-Aktivität unterbunden wird, ist nicht kritisch. Es werden im Stand der Technik eine ganze Reihe von CSF-1-Aktivität-inhibierenden Substanzen beschrieben.

Die zwei wesentlichen "Stossrichtungen" bei der Inhibierung von CSF-1-Aktivität bilden dabei die Unterdrückung der CSF-1-Aktivität selbst und die Unterdrückung der Aktivität des CSF-1-Rezeptors (siehe US 5 405 772).

Erfindungsgemäß bevorzugt als Inhibitoren der CSF-1-Aktivität sind dabei neutralisierende Antikörper gegen CSF-1 oder dessen Rezeptor. Derartige neutralisierende Antikörper (beschrieben z.B. in Weir et al., J. Bone and Mineral Research 11 (1996), 1474-1481) binden CSF-1 oder den CSF-1-Rezeptor derartig, dass eine CSF-1-Aktivität unterbunden wird bzw. nicht weitergeleitet wird.

Alternativ dazu kann CSF-1-Aktivität unter Zuhilfenahme der Antisense-Technologie unterbunden werden, bei welcher kurze Sequenzen einzelsträngiger Nukleinsäuren zur Verhinderung der Expression von CSF-1 oder dessen Rezeptor oder einem anderen Teil des Signaltransduktionsmechanismus der CSF-1-Aktivität verwendet werden. Die Antisense-Technologie ist dem Fachmann geläufig (z.B. in "Antisense Technology - A Practical Approach", Lichtenstein and Nellen (Eds.), IRL Press, Oxford University Press 1997 und "Oligonucleotides as Therapeutic Agents "Ciba Foundation Symposium 209, John Wiley & Sons 1997; hierin durch Bezugnahme inkludiert) und ist für CSF-1 oder den CSF-1-Rezeptor mit jeder geeigneten Sequenz leicht adaptierbar.

Sequenzen, die als Ganzes oder als wirksames Fragment bei der Antisense-Behandlung in Frage kommen, sind u.a. in den US-Patenten 4 847 201, 5 792 450, 5 681 719, 5 861 150, 5 104 650 und 5 725 850 beschrieben.

Weiters sind auch synthetische Inhibitoren der CSF-1-Aktivität im Rahmen der vorliegenden Erfindung einsetzbar.

Die erfindungsgemäße Inhibierung der CSF-1-Aktivität eignet sich besonders gut zur Hemmung oder Retardierung des Wachstums von soliden Tumoren.

Die erfindungsgemäße Methode hat sich als besondere effizient zur Behandlung von soliden Tumoren, ausgewählt aus der Gruppe der germinalen Tumore, der epithelialen Tumore und der Adenokarzinome. Nicht behandelbar sind maligne Erkrankungen des hämatopoetischen Systems (z.B. Leukämien).

Neben den schon erwähnten bevorzugten Inhibierungen der CSF-1-Aktivität durch Neutralisieren der Antikörper oder durch Antisense-Technologie bzw. durch chemische Inhibitoren und Kompetitoren von CSF-1 oder dessen Rezeptor können erfindungsgemäß Zellen oder Zellen des soliden Tumors genetisch so verändert werden, dass sie dem Wachstum und der Entwicklung des soliden Tumors entgegenwirken. Dabei wird mittels gentherapeutischer Methoden die Aktivität von CSF-1 oder die Aktivität des CSF-1-Rezeptors durch die induzierte Expression von genetisch veränderten, insbesondere durch Deletion von zumindest Teilen des Gens für CSF-1 oder dessen Rezeptors oder einer Mutante derselben inhibiert.

Besonders bei diesem zellulären Inhibitor, für den erfindungsgemäß sämtliche geeigneten Zelltypen in Frage kommen (ausser Keimbahn-Zellen), ist das erfindungsgemäß herzustellende Arzneimittel zur intratumoralen Verabreichung formuliert, um direkt am Ort des Tumors eingesetzt werden zu können. Dies ist auch eine bevorzugte Verabreichungvariante für die übrigen Inhibitoren.

Das erfindungsgemäße Arzneimittel kann aber auch auf andere Arten verabreicht werden, insbesondere topisch, intravenös, intraarteriell, subkutan, intraperitoneal, intrapleural, intrathekal oder in Kombination mit kationischen Lipiden.

Eine besonders bevorzugte Variante der vorliegenden Erfindung besteht wie erwähnt in der Verwendung der Antisense-Methode, d.h. einem Verfahren, bei welchem bestimmte Bereiche einer mRNA, die für CSF-1 oder dessen Rezeptor kodiert, in umgekehrter Richtung vorliegen, eingesetzt werden. Demgemäß kann die erfindungsgemäße Inhibition der CSF-1-Aktivität auch mit gentherapeutisch exprimierbaren CSF-1-Antisense-Konstrukten herbeigeführt werden.

Diese CSF-1-Antisense-Konstrukte können beispielsweise hergestellt werden, indem die folgenden Schritte durchgeführt werden:

a) Amplifizieren von CSF-1 DNA mit PCR
b) Subklonieren des PCR-Produkts von CSF-1 in einer Antisense-Orientierung
c) Einbau der in Schritt b) E.coli DNA und und
d) Isolierung des in E.coli vermehrten CSF-1-Antisense-Konstrukts.

Hiebei werden beim Amplifizieren von CSF-1-DNA mit PCR entweder geringe Mengen einer CSF-1-cDNA oder einer cDNA-Bank unter Zugabe von entsprechender Taq-DNA-Polymerase amplifiziert. Das Amplifizierungsprodukt, d.h. das PCR-Produkt von CSF-1, wird in der Folge in seiner Antisense-Orientierung in einen Vektor subkloniert, worauf die gewonnene, rekombinante DNA, d.h. die CSF-1-Antisense-Sequenz, die in den Vektor kloniert ist, durch Transformation in E.coli eingeschleust und vermehrt wird, worauf das in E.coli vermehrte CSF-1-Antisense-Konstrukt, d.h. das Plasmid, durch Standardverfahren aus den bakteriellen Zellen isoliert und der weiteren Verwendung zugeführt wird. Zur Isolierung kann beispielsweise das an sich bekannte Verfahren der alkalischen Lyse zur Plasmidisolierung eingesetzt werden. Eine nachfolgende Sequenzierung der amplifizierten und klonierten CSF-1-Antisense-Konstrukte kann durchgeführt werden. Dieses Verfahren zeichnet sich durch besondere Einfachheit und Genauigkeit aus und es ist mit diesem erfindungsgemäßen Verfahren möglich, rasch und sicher hohe Ausbeuten an spezifisch wirkenden CSF-1-Antisense-Konstrukten zu erhalten. Eine ausführliche Verfahrensführung kann hiebei dadurch beschrieben werden, dass folgende Schritte ausgeführt werden:

a) Amplifizieren von CSF-1-DNA mit PCR
b) Subklonieren des PCR-Produkts von CSF-1 in einer Antisense-Orientierung
c) Vermehrung der in Schritt b) erhaltenen CSF-1-Antisense-cDNA-Konstrukte und
d) Integration in rekombinante virale Transfervektoren
e) Vermehrung der in Schritt c) erhaltenen Konstrukte und Kotransfektion dieser gemeinsam mit Adenovirus DNA in Zellkulturzellen
f) Rekombination der CSF-1-Antisense-cDNA-Konstrukte mit Adenovirus DNA und
g) Vermehrung der Rekombinanten in Zellkulturzellen
h) Herstellung und Reinigung der rekombinanten adenoviralen CSF-1-Antisense-Konstrukte
i) und deren Einsatz in Säugerorganismen (Gentherapie von Zellkulturtumorzellen, Versuchstieren (Maus, Ratte), Einsatz bei Tumorpatienten,
j) Auswahl von CSF-1 Primärsequenzbereichen, die sich zur Oligonukleotid-Antisense-Hemmung eignen
k) Herstellung und Modifikation von nukleaseresistenten CSF-1-antisense-Oligonukleotiden
l) Einsatz dieser in Säugerorganismen (Gentherapie von Zellkulturtumorzellen, Versuchstieren (Maus, Ratte), Einsatz bei Tumorpatienten).

Die Amplifizierung von Gesamt-CSF-1 (diese Methode ist selbstverständlich auch 1:1 auf den CSF-1-Rezeptor anwendbar) oder auch Teilen desselben kann in bevorzugter Weise mit 3'-Primern bzw. 5'-Primern durchgeführt werden, wobei die Primerlänge insbesondere 15 bis 30 Nukleotide beträgt und für die Erzielung einer besonders sicheren und zielgenauen, insbesondere spezifischen Amplifizierung, bevorzugt folgende 3'-Primer

- ccagccaaga tgtggtgacc aagactgatt (Nukleotide Nr. 641-670)
- ccaagcagcg gccacccagg agcacctgcc (Nukleotide Nr. 851-880)
- aggtggaact gacagtgtag agggaattct (Nukleotide Nr. 1751-1780)
- tgcacaagct gcagttgacg tagctcgag (Nukleotide Nr. 3911-3939) bzw. 5'-Primer
- catgggtcat ctcggcgcca gagccgctct (Nukleotide Nr. 1-30)

- agccagctgc cccgtatgac cgcgccgggc (Nukleotide Nr. 91-120)
- ggagtatcac cgaggaggtg tcggagtact (Nukleotide Nr. 191-220)

eingesetzt werden.

Um eine besonders exakte und spezifische Amplifizierung zu erreichen, wird das erfindungsgemäße Verfahren bevorzugt so geführt, dass die Amplifizierung von CSF-1-DNA mit 20 bis 40 Zyklen, insbesondere 25 bis 35 Zyklen, für eine Denaturierung, Anlagerung und Extension in einer PCR-Maschine durchgeführt wird, wobei insbesondere eine programmierbare PCR-Maschine aus Gründen der Exaktheit der Verfahrensführung Verwendung findet. Hiebei wird erfindungsgemäß die Denaturierung bei 85°C bis 100°C für 20 s bis 4 min, insbesondere bei 93°C bis 98°C für 30 s bis 2 min, durchgeführt, wodurch eine vollständige, nahezu 100 %-ige Denaturierung der Proteinsequenz sichergestellt wird. Erfindungsgemäß bevorzugt erfolgt die Anlagerung bei 30°C bis 70°C für 30 s bis 4 min, insbesondere bei 37°C bis 65°C für 1 min bis 2 min, wobei gemäß dieser Verfahrensführung sichergestellt werden kann, dass die Anlagerung so vollständig wie möglich durchgeführt wird, wobei aufgrund des breiten Temperaturintervalls, in welchem dieses Verfahren durchgeführt werden kann, insbesondere auch eine energetisch günstige Verfahrensführung erzielt werden kann, da nach der Denaturierung für die Anlagerung die Temperatur nicht wie in vielen bekannten Verfahren unbedingt bis auf etwa Körpertemperatur abgesenkt werden muß. Schließlich erfolgt bevorzugt die Extension bei 65°C bis 80°C für 30 s bis 6 min, insbesondere bei 72°C bis 74°C für 1 min bis 4 min, wobei sich insbesondere aus der Gesamtheit der Verfahrensführung in der PCR-Maschine ergibt, daß trotz der Vielzahl von zur Erreichung eines vollständig und spezifisch amplifizierten Gesamt-CSF-1 oder Teilem desselben die Verfahrensdauer relativ kurz gehalten werden kann.

Zur weiteren Vereinfachung, insbesondere Vervollständigung der Verfahrensführung wird erfindungsgemäß vor den Zyklen zur Denaturierung, Anlagerung und Extension bzw. nach denselben das Verfahren bevorzugt so geführt, dass zu Beginn der Amplifizierung ein zusätzlicher Denaturierungsschritt bei ca. 95°C für ca. 2 min und am Ende der Amplifizierung eine abschließende Extension bei 72°C bis 74°C für ca. 5 bis 10 min durchgeführt wird. Durch diese zusätzliche Denaturierung zu Reaktionsbeginn wird bereits ein großer Prozentsatz der Proteine vor Durchführung der Verfahrenszyklen denaturiert, was insbesondere in den ersten Verfahrenszyklen zu vollständigerem Umsatz führt. Schließlich hat sich herausgestellt, dass durch den Einsatz einer abschließenden Extension die Produktausbeute weiter gesteigert werden kann.

Zur Subklonierung der als PCR-Produkt von CSF-1 synthetisierten cDNA wird vorzugsweise so vorgegangen, dass die als PCR-Produkt von CSF-1 synthetisierte cDNA in einen Plasmidvektor, insbesondere pCRII, subkloniert und in die MCS des pCRII-Vektors durch Inkubieren für 1 bis 24 h bei 4°C bis 25°C integriert wird. Hiebei erfolgt erst eine Subklonierung in einen Plasmidvektor, wobei sich hier bevorzugt der bekannte Vektor pCRII als geeignet erwiesen hat, welcher beispielsweise von der Firma InVitrogen käuflich erworben werden kann. Die Integrierung der cDNA in die MCS, d.h. die Multiple Cloning Site (mehrfache Klonierungsstelle) des Vektors pCRII erfolgt durch mildes Inkubieren gemäß den verschiedensten bekannten Inkubierungsverfahren, wobei sich herausgestellt hat, dass ein molares Verhältnis von Insert zu Vektor von 1:3 eine besonders sichere und vollständige Ligation ergibt. Bei der Integrierung der cDNA in den Vektor kann beispielsweise die Schnittstelle die EcoRI-Erkennungssequenz des MCS verwendet werden, wodurch eine weitere Verbesserung des erfindungsgemäßen Verfahrens erzielt werden kann.

Schließlich wurde festgestellt, dass ein besonders effizienter und sicherer Einbau der DNA in E.coli erzielt werden kann, indem bevorzugt der Einbau in E.coli durch bakterielle Transformation mittels Hitzeschock durch schockartige Erwärmung einer eisgekühlten Mischung aus E.coli-Zellen und zu transformierender DNA auf etwa 40°C bis 44°C, insbesondere 42°C, und anschließende, schnelle Abkühlung im Eisbad sowie anschließendes Inkubieren und Züchten durchgeführt wird.

Ein anderes, erfindungsgemäß ebenso bevorzugtes Verfahren besteht darin, dass der Einbau der DNA in E. coli durch Transformation von E.coli mit Plasmid-DNA durch Elektroporation bei insbesondere 25 uF, 2,5 KV und einem Widerstand von 200 Ohm und anschließendes Regenerieren, Inkubieren und Züchten der Zellkolonie durchgeführt wird.

Beide Einbauverfahren in E.coli zeichnen sich durch hohe Ausbeuten beim Anzüchten der Kolonien aus und es kann auf diese Weise mit einem einfachen Transformationsverfahren eine ausreichende Menge des erfindungsgemäßen Konstrukts für eine Weiterverwendung in der Karzinomtherapie erzielt werden. Ein weiterer Vorteil des erfindungsgemäßen Verfahrens besteht darin, dass das Konstrukt in hoher Reinheit und mit großer Selektivität erhältlich ist, sodass eine weitere Reinigung nach der Isolierung des Konstrukts nicht erforderlich ist, wodurch sowohl die Verfahrensdauer als auch die Verfahrenskosten deutlich gesenkt werden können.

Neben der Möglichkeit, CSF-1 mittels PCR aus einer bereits vorhandenen cDNA-Bank zu amplifizieren und zu isolieren, kann bevorzugt die mit PCR zu amplifizierende CSF-1 DNA durch Isolierung von Gesamt-RNA an CSF-1 exprimierenden Zellen, insbesondere Fibroblasten, oder mRNA, und nachfolgend cDNA-Synthese durch Umkehrtranskription mit PCR hergestellt werden. Derartige Klonierungsverfahren sind in allgemeiner Weise in der Technik bekannt und mussten zur Erzielung der speziellen, mit PCR zu amplifizierenden CSF-1 DNA entsprechend adaptiert und überarbeitet werden. Hiebei hat sich herausgestellt, daß die Gesamt-RNA aus CSF-exprimierenden Zellen, insbesondere Fibroblasten, in besonders bevorzugter Weise durch die Guanidinthiocyanat-Methode zur RNA-Extraktion durchgeführt werden kann, wobei zur Isolierung der alternativ eingesetzten Messenger-RNA die Oligo-dT-Zellulose-Chromatografie eingesetzt werden kann, welche eine sehr spezifische Reaktionsführung ist, bei der sehr hohe Produktausbeuten erzielt werden können. Die nach der Isolierung der Gesamt-RNA oder der Messenger-RNA erforderliche Umkehrtranskription mit PCR kann hiebei in ähnlicher Weise, wie in den erfindungsgemäßen Verfahren beschrieben, durchgeführt werden, wobei sich bei diesem Verfahren herausgestellt hat, daß die Zyklenzahl auf der PCR-Maschine geringfügig erhöht werden sollte, um vollständige bzw. selektive Produkte zu erzielen. Analoges gilt auch für die abschließende Extension, welche günstigerweise für wenigstens 10 min durchgeführt werden sollte. Bei der erfindungsgemäß vorgeschlagenen Isolierung von Gesamt-RNA oder mRNA und nachfolgender cDNA-Synthese kann jedoch im Vergleich zu dem Verfahren, bei welchem eine mRNA-Bank eingesetzt wird, ein noch spezifischeres und reineres Produkt erzielt werden, wobei dieses Produkt nur unter geringfügig erhöhtem Zeitaufwand und unter erhöhten Kosten gewonnen werden kann.

Schließlich kann, um eine weitere Verbesserung der Verfahrensführung und insbesondere eine noch höhere Spezifität bzw. Reinheit des Produkts zu erzielen, vor der Ligierung mit Adaptern eine Reinigung über Gelfiltration vorgenommen werden, wodurch das Ausgangsprodukt von kleineren, für die erfindungsgemäße Verfahrensführung nicht erforderlichen Fragmenten gesäubert wird. Auch wird die Klonierungseffizienz durch diese Verfahrensführung gesteigert werden, indem die DNA phosphoryliert wird und die Reinigung der auf diese Weise gewonnenen cDNA mittels Standard-DNA-Reinigungsprotokollen oder durch Verwendung von Affinitätschromatografie durchgeführt wird. Eine weitere Erhöhung der Ausbeute und insbesondere Verbesserung der Reinheit kann durch zusätzliche Extraktion mit TE-Puffer erreicht werden.

Gemäß einer weiteren Aufgabe zielt die Erfindung auf ein Verfahren ab, in welchem gentherapeutisch exprimierbare CSF-1-Antisense-Konstrukte hergestellt werden, wobei diese Aufgabe dadurch gelöst wird, dass gentherapeutisch exprimierbare CSF-1-Antisense-Konstrukte durch Bildung von rekombinanten, infektiösen Adenoviren durch Ausschneiden der CSF-1-cDNA aus dem Plasmidvektor und anschließendes Klonieren in einer Antisense-Orientierung in einem adenoviralen Transfervektor hergestellt werden. Hiebei wird die CSF-1-cDNA aus dem Plasmidvektor, insbesondere pCRII, mit Restriktionsenzymen herausgeschnitten und anschließend in einer Antisense-Orientierung in einen Transfervektor, der seinerseits durch Restriktionsenzyme geschnitten wurde, kloniert, worauf E.coli in an sich bekannter Weise transformiert wird und anschließend ein Screening nach rekombinanten Plasmiden durchgeführt wird. Auf diese Weise kann in hoher Ausbeute der rekombinante Transfervektor, der die integrierte CSF-1-cDNA in Antisense-Orientierung enthält, erhalten werden. In der Folge wird der rekombinante Transfervektor in adenovirale DNA eingebaut, um einen adenoviralen Transfervektor zu erzielen. Hiebei wird erfindungsgemäß bevorzugt so vorgegangen, dass die infektiösen, rekombinanten Adenoviren durch homologe Rekombination zwischen einem einen CSF-1-cDNA integriert aufweisenden Transfervektor und einem adenoviralen, genomischen Plasmid, insbesondere Ad5, gebildet werden. Dadurch, dass rekombinante, adenovirale Vektoren durch homologe Rekombination zwischen dem Transfervektor und dem adenoviralen, genomischen Plasmid, im vorliegenden Fall in der humanen Tumorzelllinie 293 erfolgend, erhalten werden, gelingt es, ein Produkt zu erzielen, welches einerseits CSF-1 in der Antisense-Orientierung aufweist, und andererseits ein replikationsdefektes Virus enthält, welches sich nur in Zellen vermehren kann, die die Defektstellen, wie beispielsweise E1A und E1B-Gene, in der trans-Stellung zur Verfügung stellen, wodurch eine selektive Vermehrung der Viren sichergestellt werden kann. Durch diese selektive Vermehrung der replikationsdefekten Viren ist ein ebenso selektiver Einsatz derselben möglich.

Die erfindungsgemäß eingesetzten, rekombinanten Ad5-Viren sind Helfer-unabhängige Viren, die in der erfindungsgemäß bevorzugt eingesetzten, humanen Zelllinie 293 vermehrt werden können.

Erfindungsgemäß können auch als Oligonukleotide CSF-1-Phosphorothioat-Antisense-Oligonukleoide (5-Propinylanaloga), CSF-1-Methylphosphonat-Antisense-Oligonukleotide, CSF-1-2'-O-Methyl-Antisense-Oligoribonukleotide oder terminal modifizierte CSF-1-Antisense-Oligonukleotide bzw. die entsprechenden Antisense-Oligonukleotide für den CSF-1-Rezeptor verwendet werden. Derartige Oligonukleotide sind im Stand der Technik für die verschiedensten Wachstumsfaktoren bekannt und werden nach Standardverfahren hergestellt.

Bei der "Antisense-Hemmtechnik" auf der Basis von genspezifischen Oligodeoxynukleotiden ist hiebei eine Modifikation des einzelsträngigen, synthetischen DNA-Moleküls notwendig, um seine Nukleaseresistenz zu erhöhen. Phosphorothioat-modifizierte Oligonukleotide weisen eine größere Stabilität im Vergleich zu den nichtmodifizierten Oligonukleotiden auf, wobei eine Substitution eines O-Atoms durch S an der Phosphodiester-Brücke erfolgt. Dadurch kann beispielsweise eine längere Wirkdauer bei niedrigeren applizierten Mengen erreicht werden. Dieser Art modifizierte Oligonukleotide weisen gegen intrazellulären Nukleaseabbau eine höhere Resistenz auf und können erfindungsgemäß als Antisense-Moleküle zur Inhibition der Genexpression und als Chemotherapeutika eingesetzt werden. Hiebei ist zu beachten, daß selbstverständlich auch die Oligonukleotide im therapeutischen Einsatz nur in gereinigter Form zur Anwendung gebracht werden dürfen, um kürzere oder fehlerhafte Addukte bzw. Synthesenebenprodukte vor der Verwendung abgetrennt zu haben. Gemäß der Erfindung können hiebei sowohl vollständig modifizierte Oligonukleotide als auch nur partiell modifizierte Phosphorthioatbrücken tragende Oligonukleotide eingesetzt werden, wobei wie erwähnt die Wirkungsweise und die Wirksamkeit der Oligonukleotide geringfügig unterschiedlich ist und beispielsweise die terminal modifizierten CSF-1-Antisense-Oligonukleotide eine erhöhte Affinität zwischen der. Zielsequenz und dem Antisense-Oligonukleotid als auch eine verbesserte Aufnahme in der Zelle, eine erhöhte Resistenz gegen einen Nukleaseabbau und eine bessere Nachweisbarkeit aufweisen. Prinzipiell ist jedoch festzuhalten, daß sämtliche Oligonukleotide in der Karzinomtherapie analog zu den CSF-1-Antisense-Konstrukten eingesetzt werden können, wobei die erfindungsgemäße Verwendung bevorzugt topisch, intravenös, intraarteriell, subkutan, intraperitonal oder in Kombination mit kationischen Lipiden erfolgt.

Die ebenfalls erfindungsgemäß hergestellten und verwendbaren, gentherapeutisch exprimierbaren CSF-1-Antisense-Konstrukte werden bevorzugt intratumoral verabreicht, da durch die intratumorale Verabreichung sichergestellt werden kann, dass das replikationsdefekte Virus zur Infektion der Tumorzellen von dem Körper an der hiefür erforderlichen Stelle herangezogen wird. Prinzipiell könnte theoretisch auch das gentherapeutisch exprimierbare CSF-1-Antisense-Konstrukt auf herkömmliche Arten, wie topisch, intravenös, intraarteriell, subkutan usw., verabreicht werden, wobei jedoch hier die Wirksamkeit deutlich eingeschränkt erscheint.

Durch die Herstellung und Verwendung von CSF-1-Antisense-Konstrukten, CSF-1-Antisense-Oligonukleotiden sowie gentherapeutisch exprimierbaren CSP-1-Antisense-Konstrukten gelingt somit die Herstellung und Verwendung von biologischen Substanzen, die das Wachstum und die Vermehrung von Karzinomzellen deutlich hemmen bzw. inhibieren, wodurch eine selektive und zielgerichtete Karzinomtherapie mit den erfindungsgemäß hergestellten Konstrukten möglich ist.

Gemäß einer besonders bevorzugten Verwendung wird erfindungsgemäß so vorgegangen, daß als CSF-1-Sequenzen von Nukleotid 1 -180 (abgeleitet aus der humanen CSF-1-Gensequenz, EMBL acc.nr. M37435, LOCUS: HUMCSDF1), insbesondere folgende 14-mere

```
ON-1CSFlas:     5 - GCCCGGCGCGGTCA-3      14-mer homolog zu den
                                          ersten 14 nt nach dem
                                          Start-Codon (ATG)
                                          (Nukleotide 120-106)


ON-2CSFlas:     5 - ACGGGGCAGCTGGC-3      14-mer homolog zu den
                                          14 nt vor dem Start-
                                          Codon (ATG)(Nukleotide
                                          105-91)

ON-3CSFlas:     5 - CGAGAGGACCCAGG-3      14-mer homolog zu den
                                          14 nt nach dem Trans-
                                          kriptionsstart der mRNA
                                          (Nukleotide 14-1)
```

eingesetzt werden.

Die Erfindung wird an Hand der nachfolgenden Beispiele auf die sie selbstverständlich nicht eingeschränkt sein soll, näher erläutert.

**Beispiel 1 :**

**Herstellung der CSF-1-cDNA-Konstrukte**

Zur Isolierung von als Ausgangsmaterial einzusetzender Gesamt-RNA aus CSF-1-exprimierenden Zellen (L929 Fibroblasten) wird die Guanidin-Thiocyanat-Methode zur RNA-Extraktion angewandt. Hiebei wird wie folgt vorgegangen:

·  Entfernung des Mediums von den L929-Fibroblasten, Zugabe von 1 ml Denaturierungslösung und Zelllyse durch Pipettieren

·  Überführen des Homogenats in 5 ml Röhrchen und Zugabe von 0,1 ml 2 M Natriumazetat (pH 4), Mischen, danach Zugabe von 1 ml wassergesättigtem Phenol, Mischen, Zugabe von 0,2 ml Chloroform/Isoamylalkohol (49:1), Mischen und Inkubieren der Suspension bei 0-4°C für 15 min

- 20 min Zentrifugieren bei 4°C und 10.000 g, die wässrige Phase in ein neues Röhrchen überführen

- Präzipitieren der RNA durch Zugabe von 1 Volumen 100% Iso propanol, Proben für 30 min auf -20°C abkühlen, dann bei 4°C für 10 min und 10.000 g zentrifugieren, überstehende Lösung verwerfen

- das oben gebildete RNA-Pellet in 0,3 ml Denaturierungslösung auflösen

- die RNA mit 0,3 ml 100% Isopropanol für 30 min bei -20°C, dann 10 min bei 4°C präzipitieren und 10.000 G zentrifugieren und die überstehende Lösung verwerfen

- Resuspendieren des RNA-Pellets in 75% Ethanol, heftig rühren und für 10-15 min bei Raumtemperatur inkubieren

- 5 min bei 10.000 g zentrifugieren, überstehende Lösung ver werfen und das RNA-Pellet im Vakuum für 5-15 min trocknen

- das RNA-Pellet in 200 μl DEPC behandeltem Wasser auflösen, Quantifizieren der RNA mittels UV-Spektrophotometrie bei 260 nm.

Amplifizieren der CSF-1-RNA mittels RT-PCR (Reverse transcriptase PCR).
1 μg der gewonnenen CSF-1-RNA in ein Mikrozentrifugenröhrchen geben und 10 min bei 70°C inkubieren, kurz zentrifugieren, danach auf Eis stellen.
Vorbereiten einer 20 μl-Reaktion durch Zugabe zu CSF-1-RNA folgender Reagenzien:

| MgCl$_2$, 25 mM | 4 μl |
|---|---|
| Reverse Transkription Puffer, 10x | 2 μl |
| dNTP-Mischung, 10 mM | 2 μl |
| Rnasin-Ribonuklease-Inhibitor | 0,5 μl |
| AMV Reverse Transkriptase | 15 Einheiten |
| Oligo(dT)-Primer | 0,5 μg |
| CSF-1-RNA | 1 μg |
| Nukleasefreies Wasser auf ein Gesamtvolumen von | 20 μl |

Danach wird die Reaktion bei 42°C für 15 min inkubiert und dann auf 99°C für 5 min erhitzt und neuerlich bei 0-5°C für 5 min inkubiert. Zur Amplifizierung wird die Lösung wie folgt verdünnt: Die Erst-Strang-cDNA-Synthese-Reaktion wird mit Nuklease-freiem Wasser auf 100 μl verdünnt und anschließend wird die 50 μl PCR-Amplifizierungs-Reaktionsmischung durch Kombination der folgenden Reagentien (Template-spezifische upstream und downstream Primer müssen hier zugegeben werden, d.h. CSF-1-spezifische Primer) vorbereitet:

```
für 5'-Primer: CSF1 sense 5'-atgaccgcgccgggc
(Nukleotide Nr. 106-120)
für 3'-Primer: CSF1 antisense 5'-cactggcagttccacctgtct
(Nukleotide Nr. 1767-1747)
```

Folgender PCR-Reaktionsansatz kam zum Einsatz: H$_2$O

| | Volumen | Endkonzentration |
|---|---|---|
| MgCl$_2$, 25 mM | 3 μl | 1,5 mM |
| 10X PCR-Puffer | 5 μl | 1x |

(fortgesetzt)

| | Volumen | Endkonzentration |
|---|---|---|
| dNTP, 10 mM | 1 µl | 200 µM von jedem |
| Upstream Primer | 5 - 50 pM | 0,1 - 1 µM |
| Downstream Primer | 5 - 50 pM | 0,1 - 1 µM |
| Taq DNA-Polymerase, 5 u/µl | 0,25 µl | 1,25 Units/50 µl |
| Erst-Strang cDNA Reak. | 10 µl | |
| Nuklease-freies H$_2$O auf ein Vol. von | 50 µl | |

Hiebei erfolgte die Zugabe der Taq-Polymerase zuletzt.

Die PCR-Maschine war mit den Zeiten und Temperaturen für Denaturierung, Anlagerung und Extension wie folgt programmiert:

Denaturierung zu Reaktionsbeginn: 95°C für 5 min. 1 Zyklus

```
Denaturierung: ca. 95°C für 1:00 min.
Anlagerung: 65°C für 1:00 min.          35 Zyklen
Extension: 72°C für 2:00 min.
```

Finale Extension: 72°C für 5 min, nach dem letzten Zyklus

Die Mischung wird auf 4°C gehalten, bis die PCR-Maschine ausgeschalten wird und die Proben entfernt werden. Zu jeder PCR-Reaktion werden 100 µl Chloroform zugegeben, gerührt, 2:00 min zentrifugiert und die obere Phase zur Weiterverarbeitung aufbewahrt. Zur Größenbestimmung des Produktes werden 10 µl des PCR-Produktes mit DNA-Größenmarkern auf ein Agarosegel aufgetragen.

Anschließend wird das PCR-Produkt wie folgt gereinigt:

· Zugabe von 250 µl Puffer PB zu 50 µl der PCR-Reaktion.
· Eine QIAquick Spin-Säule wird in ein 5 ml Zentrifugenröhrchen gegeben.
· Die Probe wird auf die Säule geladen und bei 3000 g 1 min zentrifugiert.
· Waschen: Zugabe von 0,75 ml Puffer PE und 1 min Zentrifugieren.
· Überführen der QIAquick-Säule in ein Mikrozentrifugenröhrchen. Zentrifugieren für 1 min bei 10 000 g.
· Die QIAquick-Säule in ein 1,5 ml Reaktionsgefäß geben.
· Eluierung der DNA durch Zugabe von 50 µl 10 mM Tris-Cl, pH 8,5 und Zentrifugieren für 1 min bei max. Geschwindigkeit in einer Mikrozentrifuge.

Gesammeltes Eluat: ca. 48 µl. Die DNA-Konzentration wird mittels UV-Spektrophotometrie bei 260 nm bestimmt.

Zur weiteren Umsetzung wird günstigerweise eine EcoRI-Adaptor-Ligation wie folgt durchgeführt:

| | |
|---|---|
| T4 DNA-Ligase 10X Puffer | 3 µl |
| Acetyliertes BSA, 1 mg/ml | 3 µl |
| cDNA (50 ng/µl) | 5 µl |
| Adaptoren (20-facher molarer Überschuss: 10 pmol-Adapter) | 1 µl |
| T4 DNA-Ligase (Weiss units) | 2,5 µl |
| mit Nuklease-freiem Wasser auffüllen auf | 30 µl |

Die gebildete Lösung wird über Nacht bei 15°C inkubiert, das Enzym durch Erhitzen des Reaktionsansatzes auf 70°C für 10 min inaktiviert und anschließend die Reaktion auf Eis abgekühlt.

Zur reibungsfreien Durchführung der Reaktion wird die Insert-DNA wie folgt phosphoryliert:

| Ligationsansatz | 30 μl |
| T4 PNK 10X Puffer | 4 μl |
| ATP, 0,1mM (1:100 Verdünnung in Wasser einer 10mM Stammlösung) | 2 μl |
| T4 PNK (10u/μl) | 1 μl |
| Nuklease-freies Wasser | 3 μl |
| Gesamtvolumen | 40 μl |

Die Lösung wird bei 37°C für 30 min inkubiert, anschließend wird 1 Volumen TE-gesättigtes Phenol:Chloroform zugesetzt, 30 s Rühren und 3 min mit max. Geschwindigkeit in einer Mikrozentrifuge zentrifugieren, die obere wässrige Phase in ein neues Röhrchen übertragen. Der überschüssige Adaptor wird dann wie folgt entfernt:

250 μl Puffer PB werden zu der Phosphorylierungsreaktion zugegeben, eine QIAquick Spin-Säule wird in ein 5 ml Zentrifugenröhrchen eingebracht, die Probe auf die Säule geladen und bei 3000 g 1 min zentrifugiert, anschließend durch Zugabe von 0,75 ml Puffer PE gewaschen und neuerlich 1 min zentrifugiert, die QIAquick-Säule in ein Mikrozentrifugenröhrchen überführt und für 1 min bei 10 000 g zentrifugiert, danach wird die QIAquick-Säule in ein 1,5 ml Reaktionsgefäß gegeben und die DNA durch Zugabe von 50 μl 10 mM Tris-Cl, pH 8,5, und Zentrifugieren für 1 min bei max. Geschwindigkeit in einer Mikrozentrifuge eluiert. Gesammeltes Eluat: ca. 48 μl.

Als nächster Schritt wird die cDNA durch Ethanol-Präzipitation wie folgt konzentriert:

Die DNA wird mit 0,5 Volumina 7,5M Ammoniumacetat und 2,5 Volumina kaltem (-20°C) 100% Ethanol, gemischt, Mischen und Placieren bei -70°C für 30 min, anschließend bei max. Geschwindigkeit in einer Mikrozentrifuge für 15 min zentrifugiert und die überstehende Lösung entfernt, das gebildete Pellet mit 1 ml kaltem (-20°C) 70% Ethanol gewaschen und in einer Mikrozentrifuge mit max. Geschwindigkeit für 5 min zentrifugiert, die überstehende Lösung wird entfernt, das Pellet kurz im Vakuum getrocknet, das Sediment in 50 μl TE-Puffer für die Weiterbearbeitung neuerlich suspendiert. Die DNA-Konzentration wird mittels UV-Spektrophotometrie bei 260 nm bestimmt.

Der pCRII-Vektor wird dann einer Phosphatase-Behandlung unterzogen, wobei der Vektor vor der Phosphatasebehandlung durch einen Restriktionsschnitt mit EcoRI wie folgt linearisiert wird:

Restriktionsansatz:

1 μg pCRII DNA
2 μl 10x EcoRI-Puffer
2 Units EcoRI
Mit Wasser auf ein Gesamtvolumen von 20 μl auffüllen.
2 Stunden bei 37°C inkubieren.

Dephosphorylierung der Vektor-DNA:

Zugabe von 1/10 Volumen 10x Dephosphorylierungspuffer. Inkubation nach Zugabe von 1 Einheit alkalischer Phosphatase für 60 min bei 37°C. Inaktivierung der alkalischen Phosphatase durch 15 min Erhitzen auf 65°C.

Danach wird die synthetisierte cDNA in die EcoRI-Schnittstelle des Vektors pCRII kloniert.

Ligationsansatz:

100 ng Vektor-DNA
50 ng CSF1-cDNA
1 μl T4 DNA-Ligase (1 Weiss Einheit)
1,5 μl T4 DNA-Ligase 10x Puffer

mit Nuklease-freiem Wasser auf 15 μl auffüllen, der Reaktionsansatz wird 3 h bei Raumtemperatur inkubiert und das pCRII-CSF-1-rekombinante Plasmid gewonnen.

Einbau der DNA in E. coli:

Das Plasmid pCRII-CSF-1 wird durch Transformation in E.coli eingeschleust und wie folgt vermehrt:

Transformation von Bakterien durch Elektroporation

- 100 μl elektrokompetente E.coli werden mit dem halben Volumen des Ligationsansatzes (7,5 μl) in Küvetten auf Eis gemischt
- Elektroporation: 25 μF, 2,5 kV, 200 Ω
- Zugabe von 1 ml SOC-Medium zum Regenerieren der Zellen, Transfer der Zellen in ein Röhrchen und 1 h Inkubation bei 37°C, danach Ausplattieren auf Ampicillin-Selektionsplatten und Anzüchten der Kolonien über Nacht bei 37°C.

Isolierung des Plasmids:

Eine Einzelkolonie wird von der Selektionsplatte gepickt und in 3 ml LB mit Ampicillin für 8 h bei 37°C unter starkem Schütteln inkubiert, mit 1/500 in 100 ml LB-Medium verdünnt, bei 37°C für 12 h unter starkem Schütteln wachsen gelassen. Danach werden die Bakterien durch Zentrifugation bei 6000 g für 15 min bei 4°C geerntet, die bakteriellen Pellets in 10 ml Puffer P1 gelöst, 10 ml Puffer P2 zugegeben, sorgfältig gemischt und 5 min bei Raumtemperatur inkubiert, anschließend Zugabe von 10 ml eisgekühltem Puffer P3 und sofort vorsichtiges Mischen und auf Eis 20 min Inkubieren, Zentrifugieren bei 20 000 g für 30 min bei 4°C. Die überstehende Lösung wird noch einmal bei 20 000 g für 15 min bei 4°C Zentrifugiert und auf eine equilibrierte QIAGEN-500-Säule mit 10 ml Puffer QBT überführt. Nach dem Waschen der Säule mit 2x30 ml Puffer QC wird die DNA mit 15 ml Puffer QF eluiert und durch Zugabe von 10,5 ml Isopropanol (Raumtemperatur) zu der eluierten DNA präzipitiert. Nach Mischen und sofortiger Zentrifugation bei 15 000 g für 30 min bei 4°C wird die überstehende Lösung entfernt, das DNA-Pellet mit 5 ml 70% Ethanol (Raumtemperatur) gewaschen, bei 15 000 g für 10 min zentrifugiert und die überstehende Lösung entfernt. Das gebildete Pellet wird für 5 min lufttrocknen gelassen und die DNA in 100 μl TE, pH 8,0, aufgelöst. Die DNA-Konzentration wird mittels UV-Spektrophotometrie bei 260 nm bestimmt.

Schließlich werden die Sequenzen aller amplifizierten und klonierten CSF-1-Konstrukte durch Sequenzierung nach der Standardmethode von Sanger (Kettenabbruchmethode) bestimmt. Die CSF-1-Konstrukte können nun als solche verwendet werden oder in pharmazeutisch verträgliche Formulierungen weiterverarbeitet werden.

**Beispiel 2 :**

**Herstellung von gentherapeutisch exprimierbaren CSF-1-Antisense-Konstrukten**

Herstellung von rekombinanten infektiösen Adenoviren

Die CSF-1-cDNA wird aus dem Plasmid pCRII-CSF-1 von Beispiel 1 herausgeschnitten und danach in einer Antisense-Orientierung in den adenoviralen Transfervektor kloniert:

Das Insert wird durch einen Restriktionsschnitt mit EcoRI wie folgt herausgeschnitten:

Restriktionsansatz:

1 μg pCRII-CSF1-DNA
2 μl 10x EcoRI-Puffer
2 Units EcoRI
Mit Wasser auf ein Gesamtvolumen von 20 μl auffüllen.
2 h bei 37°C inkubieren.

Anschließend werden stumpfe Enden durch eine Auffüllreaktion mit Klenow-Enzym unter Zusatz folgender Reagenzien: 2 μl 10x NTB; 1 μl 1mM dNTP; 1 Einheit Klenow, bei 37°C für 15 min inkubiert und auf 65°C für 5-10 min zur Inaktivierung des Klenow-Enzyms erhitzt, gebildet.

Danach wird der Transfervektor pQBI-AdCMV5BFP mit dem Restriktionsenzym BglII geschnitten:

Restriktionsansatz:

1 μg Transfervektor DNA
2 μl 10x Puffer M
2 Units BglII

Mit Wasser wird auf ein Gesamtvolumen von 20 µl aufgefüllt und 2 h bei 37°C inkubiert, die gebildeten Fragmente auf einem 1% TAE-Agarosegel aufgetrennt, das 1641 bp große CSF-1-Fragment sowie der Transfervektor wird wie folgt gesondert aus dem Agarosegel gereinigt:

Ausschneiden des jeweiligen DNA-Fragmentes aus dem Agarosegel mit einem Skalpell, Wiegen des Gelstückes und Zugabe von 3 Volumen des Puffers QG zu 1 Volume Gel, anschließend Inkubation bei 50°C für 10 min. Während der Inkubation wird alle 2 min gerührt, kontrolliert ob die Farbe des Gemisches gelb ist, anschließend Zugabe von 1 Gelvolumen Isopropanol zur Probe, Vermischen. Placieren einer QIAquick Spin-Säule in ein 2 ml Reaktionsgefäß und Auftragen der Probe auf die Säule und Zentrifugieren für 1 min. Säule in ein neues Reaktionsgefäß geben. Waschen durch Auftragen von 0,75 ml Puffer PE auf die Säule und Zentrifugieren für 1 min, danach Entfernen des Durchflusses und Zentrifugieren der Säule für 1 min bei 10 000 g.

Elution der DNA: Zugabe von 50 µl von 10 mM Tris-Cl, pH 8,5 und 1 min bei max. Geschwindigkeit zentrifugieren. Anschließend erfolgt eine Ligation des CSF-1-cDNA in den Transfervektor pQBI-AdCMV5BFP, nämlich:

Das gereinigte CSF-1-Fragment wird in den linearisierten Transfervektor wie folgt kloniert.

Ligationsansatz:

> 200 ng Transfervektor-DNA
> 100 ng CSF-1-cDNA
> 1 µl T4 DNA-Ligase (1 Weiss Einheit)
> 1,5 µl T4 DNA-Ligase 10x Puffer

mit Nuklease-freiem Wasser auf 15 µl auffüllen
Inkubieren des Reaktionsansatzes 6 h bei Raumtemperatur.

Die anschließende Transformation von Bakterien durch Elektroporation gelingt wie folgt:

100 µl elektrokompetente E.coli werden mit dem halben Volumen des Ligationsansatzes (7,5 µl) in Küvetten unter Eiskühlung gemischt und die Elektroporation bei 25 µF, 2,5 kV, 200 Ω ausgeführt, zum Regenerieren der Zellen werden 1 ml SOC-Medium zugegeben, Transfer der Zellen in ein Röhrchen und 1 h Inkubation bei 37°C, danach Ausplattieren auf Ampicillin-Selektionsplatten und Anzüchten der Kolonien über Nacht bei 37°C.

Das Plasmid wird anschließend wie folgt isoliert:

Eine Einzelkolonie wird von der Selektionsplatte geholt und in 3 ml LB mit Ampicillin für 8 h bei 37°C unter starkem Schütteln inkubiert, die Startkultur wird 1/500 in 100 ml LB-Medium verdünnt und bei 37°C für 12 h unter starkem Schütteln wachsen gelassen, die Bakterien werden durch Zentrifugation bei 6000 g für 15 min bei 4°C geerntet, das bakterielle Pellet in 10 ml Puffer P1 gelöst, 10 ml Puffer P2 zugegeben, gemischt und 5 min bei Raumtemperatur inkubiert, 10 ml eisgekühltem Puffer P3 zugegeben und sofort vorsichtig gemischt und auf Eis 20 min inkubiert, bei 20 000 g für 30 min bei 4°C inkubiert, die überstehende Lösung wird noch einmal bei 20 000 g für 15 min bei 4°C zentrifugiert und die überstehende Lösung auf eine mit 10 ml Puffer QBT equilibrierte QIAGEN-500-Säule überführt, die Säule mit 2x30 ml Puffer Qc gewaschen, die DNA mit 15 ml Puffer QF eluiert und durch Zugabe von 10,5 ml Isopropanol (Raumtemperatur) zu der eluierten DNA ausgefällt, gemischt und sofort bei 15 000 g für 30 min bei 4°C zentrifugiert, die überstehende Lösung wird entfernt. Das DNA-Pellet mit 5 ml 70% Ethanol (Raumtemperatur) wird gewaschen und bei 15 000 g für 10 min zentrifugiert und die überstehende Lösung entfernt, das Pellet wird 5 min luftgetrocknet und die DNA in 100 µl TE, pH 8,0, aufgelöst. Die DNA-Konzentration wird mittels UV-Spektrophotometrie bei 260 nm bestimmt. Die Auftrennung der Fragmente erfolgt auf einem 1% TAE-Agarosegel, worauf der Transfervektor aus dem Agarosegel wie folgt extrahiert und gereinigt wird:

Ausschneiden des linearisieren Vektors aus dem Agarosegel mit einem Skalpell, Abwägen des Gelstückes und Zugabe von 3 Volumen des Puffers QG zu 1 Volumen Gel, Inkubation bei 50°C für 10 min, wobei alle 2 min gerührt und kontrolliert wird, ob die Farbe des Gemisches gelb ist. Anschließend Zugabe von 1 Gelvolumen Isopropanol zur Probe, Mischen, Anordnen einer QIAquick Spin-Säule in ein 2 ml Reaktionsgefäß und Auftragen der Probe auf die Säule und Zentrifugieren für 1 min. Säule in ein neues Reaktionsgefäß geben, Waschen durch Auftragen von 0,75 ml Puffer PE auf die Säule und Zentrifugieren für 1 min. Entfernen des Durchflusses und Zentrifugieren der Säule für 1 min bei 10 000 g. Elution der DNA: Zugabe von 50 µl von 10 mM Tris-Cl, pH 8, 5, und 1 min bei max. Geschwindigkeit zentrifugieren. Ausbeute: ca. 48 µl.

Anschließend erfolgt die Kotransfektion des linearisierten rekombinanten Transfervektors (pAdCMV5-CSF-BFP) mit der viralen DNA (AD5CMVlacZE1/E3) in 293 Zellen mittels der Kalzium-Phosphat-Methode wie folgt:

Zugabe von 0,005 Volumen 2 mg/ml Carrier DNA zu 1xHEBS, Mischen durch Rühren für 1 min. Aliquotieren von 2 ml HEBS plus Carrier-DNA in ein steriles, klares Plastikreaktionsgefäß, Zugabe von 20 μg des linearisierten rekombinanten Transfervektors (pAdCMV5-CSF-BFP) und 20 μg der viralen DNA zu diesem Reaktionsgefäß und vorsichtig Schütteln, danach langsam 0,1 ml 2,5 M CaCl$_2$ zugeben, vorsichtig mischen und 25 min bei Raumtemperatur inkubieren. Zugabe von 0,5 ml DNA Suspension zu einer 60 mm-Zellkulturschale mit 293 Zellen, ohne das Wachstumsmedium zu entfernen, 5 Stunden bei 37°C in einem CO$_2$-Inkubator inkubieren, Entfernen des Mediums und Zugabe von 10 ml MEMF11-Agarose (vorher auf 44°C equilibriert). Nach Festwerden der Agarose, Inkubation bei 37°C. Plaques erscheinen nach 5-14 Tagen. Zum Screenen der Adenovirus Plaque Isolate werden die Plaques von der transfektierten Kultur durch Ausstechen mittels einer sterilen Pasteurpipette isoliert und in Reaktionsgefäße mit 0,5 ml sterilen PBS plus 10% Glyzerin transferiert. Bis zur Verwendung auf -70°C lagern. Anschließend Entfernen des Mediums von zu 80% konfluenten 293 Zellen in 60 mm Schalten und Zugabe von 0,2 ml Virus (Agar Suspension). Absorbieren bei Raumtemperatur für 30 min. Zugabe von komplettem MEMF11 + 5% Horse-Serum und Inkubation bei 37°C. Virusernte und Extraktion der infizierten zellulären DNA, wenn die meisten Zellen abgelöst sind. Vorsichtig 4 ml Medium mit einer Pipette entfernen und in ein Reaktionsgefäß mit 0,5 ml sterilem Glyzerin geben. Diese Virenkandidaten werden bei -70°C gelagert. Entfernen des verbleibenden Mediums von der Schale. Zur DNA-Extraktion aus den infizierten Zellen, Zugabe von 0,5 ml Pronase-Lösung und Inkubation bei 37°C für 10 h. Transfer des Lysates in ein 1,5 ml Reaktionsgefäß, und 1x mit Puffer-gesättigtem Phenol extrahieren, 10 min Zentrifugieren und Sammeln der oberen, wässrigen Phase und Transfer in ein neues Gefäß, Zugabe von 1 ml Ethanol zum Präzipitieren der DNA. Sorgfältig mischen. 10 min bei 14 000 rpm Zentrifugieren, Absaugen der überstehenden Lösung, Waschen des Pellets mit 70% Ethanol, 5 min Zentrifugieren, Überstand absaugen und Pellet lufttrocknen lassen. Auflösen der DNA in 50 μl 0,1 xSSC und mit 5 μl einen Restriktionsschnitt mit HindIII durchführen (1 unit über Nacht). Auftragen der verdauten Probe auf ein 1% Agarosegel mit Ethidiumbromid. Virale DNA-Banden sind unter UV-Licht danach leicht sichtbar, über einer Hintergrundschmiere von zellulärer DNA. Verifizieren von rekombinanten Viruskandidaten durch weitere diagnostische Resktriktionsenzymschnitte, sowie durch Kontrolle der Expression des BFP (Blue Fluorescent Protein) unter dem Fluoreszenzmikroskop. Korrekte Rekombinante werden durch 2 weitere Runden mittels Plaque-Reinigung weiter gereinigt und gescreent, bevor ein High-Titer-Stock hergestellt wird.

Anschließend werden zur Reinigung und Titration von rekombinanten Adenoviren (AD5CMV-CSF) wie folgt Plaque Assays durchgeführt:

Absaugen des Mediums von konfluenten 293 Zellen in 60 mm-Schalen. Zugabe von 0,2 ml Virus ($10^{-3}$-$10^{-6}$-Verdünnungen einer Agar-Suspension in PBS für Plaque-Reinigung oder $10^{-3}$-$10^{-9}$-Verdünnungen der Stammlösung für die Titration). Absorbieren des Virus für 40 min bei Raumtemperatur. Zugabe von 10 ml MEMF11-Agarose-Overlay, Abkühlen, und Inkubieren bei 37°C. Plaques werden nach 7 und nach 10 Tagen für die Titration gezählt. Plaque Reinigung: Isolierung der Plaques wie oben beschrieben.

Schließlich werden High-Titer Virale Stammlösungen von Monolayer--Zellen wie folgt hergestellt:

Zehn 150 mm-Schalen werden mit 293 Zellen angesetzt und bei Erreichen einer Konfluenz von 80% infiziert, danach wird zur Herstellung eines High-Titer-Stocks das Medium von den 293 Zellen entfernt und mit einer multiplicity of infection (MOI) von 1-10 PFU pro Zelle (1 ml Virus-Suspension pro 150 mm-Schale) infiziert, für 40 min absorbiert und dann Zugabe von MEMF11 + 5% Horse-Serum, Inkubieren bei 37°C und tägliche Kontrolle auf Anzeichen des zytopathischen Effekts. Wenn der zytopathische Effekt fast vollständig ist, Ernte durch Abschaben der Zellen von der Schale, Kombination der Zellen plus Medium und Zentrifugation bei 800 g für 15 min. Absaugen des Mediums und Resuspendieren des Zellpellets in 2 ml PBS + 10% Glyzerin pro 150 mm-Schale. Weitere Reinigung der Viruslösung durch CsCl-Dichtegradienten-Zentrifugation, nachfolgend Dialyse gegen 10 mM Tris-HCl, pH 8,0. Zugabe von sterilem Glyzerin zu einer finalen Konzentration von 10% und Lagerung bei -70°C.

Es werden so High Titer-rekombinante infektiöse adenovirale CSF-1-Antisense-Konstrukte, die direkt zur Gentherapie eingesetzt werden können, gewonnen.

**Beispiel 3:**

**Einsatz der rekombinanten infektiösen adenoviralen CSF-1Antisense-Konstrukte bei der Infektion von Zellen**

Getestete Zellsysteme:

    Lewis Lung-Karzinom-Zellen
    Dickdarmkarzinom-Zellen
    Mammakarzinom-Zellen
    Germinale Tumorzellen

Durchführung der Infektion:

Zu zell-Monolayern in 60 mm-Schalen mit 80%iger Konfluenz werden 0,5 ml Viruslösung (AD5CMV-CSF) unterschiedlicher MOI (multiplicities of infection) zugegeben und für 30 min bei Raumtemperatur inkubiert.

Die Kontrollinfektionen erfolgt hiebei mit: AD5CMVlacZ E1/E3 (Adenovirus ohne CSF-Insert), AdenoGFP (Adenovirus mit GFP), Mock-Infektion (Kulturmedium). Zugabe von 6 ml Medium und Inkubation der Schalen im $CO_2$-Inkubator (37°C, 5% $CO_2$).

Die Infektion zeigte folgende Wirkungen:

CSF-1-Genexpression in den Wirtszellen war deutlich herabgesetzt (Reduktion der mRNA (CSF-1-Protein) Spiegel in allen transfektierten Zellen auf < 30% im Vergleich zu nicht behandelten Wildtyp-Zellen.

Die Gefäßneubildung gehemmt, antionkogene Effekte (retardiertes Zellwachstum).

Bei Analyse des Zellzyklus wurde Auslösung von Apoptose festgestellt.

Bei der viralen Transfektion (Adenoviren) von Mäusen erfolgte eine intratumorale Verabreichung von $1\times10^9$ - $5\times10^{10}$ PFU in einem Volumen von max. 0,5 ml.

Eine intratracheale Administration erfolgte durch Verabreichung von bis zu $2\times10^9$ PFU des rekombinanten Virus in einem Volumen von 23-35 µl, wobei insbesondere bei der intratumoralen Verabreichung eine fast vollständige Rückbildung der Tumoren beobachtet werden konnte.

**Beispiel 4 :**

**Herstellung von CSF-1 spezifischen Oliqonukleotiden und Verwendung derselben**

Synthese eines CSF-1 spezifischen Oligonukleotides

Die Synthese des CSF-1-spezifischen Oligonukleotides 5'-GCCCGGCGCGGTCA-3' (14-mer, homolog zu den ersten 14 Nukleotiden der CSF-1-cDNA-Primärsequenz nach dem Start Codon (ATG) von Basenpaar 120-106) erfolgt auf einem automatisierten Oligonukleotid-Synthesizer, der auf der Phosphoramidit Methode basiert. Die Synthese wird in der 3'-5'-Orientierung der vorgegeben Nukleotidsequenz durchgeführt und nach Beendigung der Synthese wird die Säule mit dem gebundenen Oligonukleotid zuerst mit 3 ml konzentriertem $NH_3$ gewaschen. Der Vorgang wird mehrmals wiederholt, damit die Säule komplett mit $NH_3$ durchdrungen ist. Die Säule wird ca. 2 h bei RT mit $NH_3$ inkubiert (mehrmals gespült) und abschließend gewonnen. In einem Reaktionsgefäß wird die Lösung gut verschlossen für 16 h auf 55°C erhitzt. In einem Rotationsverdampfer wird das $NH_3$ entfernt (30 µl Triethylamin werden zugesetzt um eine Detritylierung zu verhindern).

Herstellung von Phosphorothioat-Oligonukleotiden mit dem Beaucage-Reagens:

Die Sulfurierung wird mit 240 µl einer 0,05 M Lösung von 3H-1,2-Benzodithiol-3-on-1,1-Dioxid (Pharmacia, Sigma) direkt auf der Säule, noch vor dem Cappingschritt durchgeführt, wozu ein passender Reaktionskolben mit einer Mischung von 12 ml Dichlordimethylsilan und 200 ml Dichlormethan gefüllt wird, worauf nach 5 min die Lösung entfernt und der Kolben mit Methanol ausgespült wird. Der Kolben wird anschließend über Nacht bei 110°C getrocknet und in einem Exsiccator abgekühlt. 0,5 g (2,5 mmol) des Beaucage's-Reagens werden in 50 ml trockenem Acetonitril gelöst und in den Reaktionskolben gefüllt. Der Reaktionskolben wird an den Oligosynthesizer angeschlossen und über die Säule gepumpt (2x).

Analytische reverse-Phase HPLC

(Säule: C-18 an Silika gebundene Phase mit sphärischen Partikeln (5 µm, 300 A Porengröße), zusätzlich Nucleosil 100-5 C-18, 100 mm x 4 mm)

Das ungereinigte Oligonukleotid (eingedampftes Sediment) wird in 300 µl 0,1 M Triethylammoniumacetatpuffer, pH 7, aufgenommen.

Ein UV-Monitor wird auf 260 nm gesetzt und die Flußrate auf 1 ml/min eingestellt. Es wird ein Puffergradient von 0 - 50% 1 von einem 0,1 M Triethylammoniumacetatpuffer, pH 7, in 80 % Azetonitril über 50 min gefahren. Anschließend wird derselbe Puffer in 5 min von 50 - 100% gesteigert. Eine kleine Probeportion von 5 µl des ungereinigten Oligonukleotids (2 - 5% der Gesamtmenge) wird auf die Säule aufgetragen und die Absorption bei 260 nm aufgezeichnet.

Präparative reverse-Phase HPLC

Es wird dieselbe Anordnung wie bei der Synthese eines CSF-1-spezifischen Oligonukleotids verwendet, nur wird die Gesamtmenge von 300 µl Oligonukleotidlösung auf die Säule aufgetragen und die Absorption bei 260 nm verfolgt und die zentralen Teile der eluierten Peaks werden aufgesammelt, die vereinigten Proben werden im Rotationsverdampfer sedimentiert, 1 ml 80 % Essigsäure wird zu den eingetrockneten Proben gegeben und 1 h bei RT inkubiert und die Proben werden neuerlich im Rotationsverdampfer sedimentiert. Das Pellet wird in 1 ml $H_2O$ dest./ster. gelöst, 2 mal mit DMT-OH und 1 x mit Ethylazetat extrahiert. Die Proben werden im Rotationsverdampfer eingetrocknet und sedimentiert. Der Niederschlag wird in einem bestimmten Volumen $H_2O$ dest./ster. gelöst und die Extinktion bei 260 nm gemessen, um die Menge zu bestimmen (1: 100 Verdünnung, Berücksichtigung des sequenzabhängigen Extinktionskoeffizienten).

Verwendung von CSF-1-Antisense-Phosphorothioat-Oligonukleotiden

Die hergestellten CSF-1-Antisense-Phosphorothioat-modifizierten Oligonukleotide werden als wasserlösliche Reinsubstanz (HPLC gereinigt) und in PBS gelöst auf verschiedenen Wegen appliziert. Untersucht wurden die systemische Gabe mittels intravenöser Injektion, die intraarterielle Gabe, wobei das zuführende organspezifische Gefäß als Arterie der Wahl erachtet wird, um möglichst zielnah die Substanz verabreichen zu können. Abhängig von der Lokalisierung des Tumors, wurden andere Wege, z.B. topische oder intraperitoneale Gabe untersucht. Weiters können "Osmotic Mini Pumps" als Reservoirs dienen (v.a. bei Mäusen als Versuchstiere), in die das CSF-1-Antisense gefüllt und durch subkutane oder intravenöse Implantation verabreicht wird. Der Vorteil dieser Methode liegt in der einfachen und verlässlichen Applikationsart. Weiters haben die Pumpen den Vorteil, dass sie einmal implantiert, bis zu 4 Wochen die Applikation von konstanten Raten garantieren. Die Dosis der zu verabreichenden CSF-1-Antisense-Oligonukleotide liegt hiebei im Milligramm-Bereich. So wurden in einer antionkogenen Therapie an Mäusen Dosen von 0,1-20 mg/kg Körpergewicht/Tag eingesetzt. Ratten mit 200 - 350 g Körpergewicht erhalten jeweils 100 µl Gaben intravenös in einer Konzentration von 0,1 - 1 µg/ml. Im Humansystem ist eine Dosierung 0,05 mg/kg/Stunde angebracht, da bei dieser Dosierung noch keine toxischen Effekte auf Grund der Verabreichung modifizierter Oligonukleotide alleine auftreten. CSF-1-Antisense-Oligonukleotid-Behandlungsschemata sollten für mindestens 2 Wochen kontinuierlich eingehalten werden.

**Patentansprüche**

1.    Verwendung von Inhibitoren von CSF-1-Aktivität zur Herstellung eines Arzneimittels zur Behandlung von Tumorerkrankungen.

2.    Verwendung nach Anspruch 1, **dadurch gekennzeichnet, dass** als Inhibitor der CSF-1-Aktivität ein neutralisierender Antikörper gegen CSF-1 oder dessen Rezeptor eingesetzt wird.

3.    Verwendung nach Anspruch 1 oder 2, **dadurch gekennzeichnet, dass** als Inhibitor der CSF-1-Aktivität eine Antisense-Nukleinsäure gegen CSF-1 oder dessen Rezeptor eingesetzt wird.

4.    Verwendung nach einem der Ansprüche 1 bis 3, **dadurch gekennzeichnet, dass** das Arzneimittel zur Hemmung des Wachstums von soliden Tumoren hergestellt wird.

5.    Verwendung nach einem der Ansprüche 1 bis 4, **dadurch gekennzeichnet, dass** das Arzneimittel zur Retardierung von malignen Tumor-Erkrankungen hergestellt wird

6.    Verwendung nach einem der Ansprüche 1 bis 5, **dadurch gekennzeichnet, dass** als Inhibitor der CSF-1-Aktivität eine genetisch veränderte Zelle eingesetzt wird, bei welcher die Aktivität von CSF-1 oder dessen Rezeptors durch die genetische Veränderung, insbesondere durch Deletion von zumindest von Teilen des Gens für CSF-1 oder dessen Rezeptor, inhibiert ist.

7.    Verwendung nach einem der Ansprüche 1 bis 6, **dadurch gekennzeichnet**, das das Arzneimittel zur intratumoralen Verabreichung formuliert ist.

8.    Verwendung nach einem der Ansprüche 1 bis 7, **dadurch gekennzeichnet, dass** das Arzneimittel zur Behandlung von soliden Tumoren, ausgewählt aus der Gruppe der germinalen Tumoren, epithelialen Tumoren und Adenokarzinomen, hergestellt wird.

**Claims**

1. The use of inhibitors of CSF-1 activity for preparing a medicament for the treatment of tumor diseases.

2. The use according to claim 1, **characterized in that** a neutralizing antibody against CSF-1 or against a receptor thereof is utilized as inhibitor of CSF-1 activity.

3. The use according to claim 1 or 2, **characterized in that** an antisense nucleic acid against CSF-1 or a receptor thereof is utilized as inhibitor of CSF-1 activity.

4. The use according to any one of claims 1 to 3, **characterized in that** the medicament is prepared to inhibit growth of solid tumors.

5. The use according to any one of claims 1 to 4, **characterized in that** the medicament is prepared to retard malignant tumor diseases.

6. The use according to any one of claims 1 to 5, **characterized in that** a genetically altered cell, in which the activity of CSF-1 or a receptor thereof is inhibited by a genetic alteration, in particular by deletion of at least parts of the gene for CSF-1 or the receptor thereof, is utilized as inhibitor of CSF-1 activity.

7. The use according to any one of claims 1 to 6, **characterized in that** the medicament is formulated for intra-tumoral administration.

8. The use according to any one of claims 1 to 7, **characterized in that** the medicament is prepared for treating solid tumors selected from the group consisting of germinal tumors, epithelial tumors and adenocarcinomas.

**Revendications**

1. Utilisation d'inhibiteurs de l'activité du CSF-1 pour la fabrication d'un médicament destiné à traiter des maladies tumorales.

2. Utilisation selon la revendication 1, **caractérisée en ce que** l'on a utilise un anticorps neutralisant dirigé contre le CSF-1 ou son récepteur comme inhibiteur de l'activité du CSF-1.

3. Utilisation selon la revendication 1 ou 2, **caractérisée en ce que** l'on utilise un acide nucléique antisens dirigé contre le CSF-1 ou son récepteur comme inhibiteur de l'activité du CSF-1.

4. Utilisation selon l'une des revendications 1 à 3, **caractérisée en ce que** le médicament est fabriqué pour freiner la croissance des tumeurs solides.

5. Utilisation selon l'une des revendications 1 à 4, **caractérisée en ce que** le médicament est fabriqué pour retarder l'apparition de maladies tumorales malignes.

6. Utilisation selon l'une des revendications 1 à 5, **caractérisée en ce que** l'on utilise une cellule génétiquement modifiée comme inhibiteur de l'activité du CSF-1, dans laquelle l'activité du CSF-1 ou de son récepteur est inhibée par modification génétique, en particulier par délétion d'au moins certaines parties du gène du CSF-1 ou de son récepteur.

7. Utilisation selon l'une des revendications 1 à 6, **caractérisée en ce que** le médicament est formulé pour une administration intratumorale.

8. Utilisation selon l'une des revendications 1 à 7, **caractérisée en ce que** le médicament est fabriqué pour le traitement de tumeurs solides choisies dans le groupe des tumeurs germinales, des tumeurs épithéliales et des adénocarcinomes.